# DEMANDE DE BREVET EUROPEEN

(11) **EP 2 662 029 A2**
(43) Date de publication de la demande: **13.11.2013**
(21) Numéro de dépôt: 13166760.2
(22) Date de dépôt: 07.05.2013
(51) Int. Cl.: A61B 10/00

(54) **Procédé et support de quantification des exsudats d'une plaie**

(30) Priorité: 10.05.2012 FR 1254252
(71) Demandeur: Viseux de Potter, Philippe, 59251 Allennes-Les-Marais (FR)
(72) Inventeur: Viseux de Potter, Philippe, 59251 Allennes-Les-Marais (FR)
(74) Mandataire: Roman, Alexis

(57) **Abrégé**

L'invention concerne un procédé de quantification des exsudats s'écoulant d'une plaie pendant une période déterminée, de préférence pendant une période de 24 heures, ledit procédé comprenant les étapes suivantes :
a) placer un support de quantification (1) des exsudats transparent ou semi-transparent, et comportant un abaque (2) défini par une pluralité de zones (Z1, Z2,... Zn) différentes, la surface de chacune desdites zones correspondant à une quantité déterminée d'exsudats susceptible de s'être écoulée de ladite plaie pendant ladite période, sur la tache (4, 4') formée par lesdits exsudats sur le pansement absorbant (3) ayant servi à protéger ladite plaie pendant ladite période, de manière à ce que le contour de ladite tache (4, 4') s'inscrive au moins dans la zone de l'abaque (2) qui a la plus petite surface,
b) déduire la quantité d'exsudats susceptible de s'être écoulée de la plaie pendant ladite période en déterminant la zone qui a la plus grande surface dans laquelle ladite tache (4, 4') est inscrite.

## Description

### Domaine technique de l'invention.

La présente invention a pour objet un procédé de quantification des exsudats s'écoulant d'une plaie pendant une période donnée. Elle concerne également un support de quantification des exsudats ainsi que son utilisation pour l'estimation de la quantité d'exsudats d'une plaie. Elle vise également un ensemble comportant un pansement absorbant et un support de quantification selon l'invention.

Elle se rapporte au domaine technique de recueil d'exsudats et des accessoires pour pansements absorbants.

### État de la technique.

Par « exsudat » ou « exsudats » on entend un fluide produit par les plaies aiguës ou chroniques, les fistules ou toute autre plaie. Il provient du sang par extravasation à partir des vaisseaux au niveau du lit de la plaie. En général, l'exsudat est produit en grande quantité juste après la formation de la plaie (phase de détersion) et a tendance à diminuer au fur et à mesure que la plaie cicatrise. Il doit néanmoins persister jusqu'à cicatrisation.

L'exsudat contient en proportion variable diverses substances telles que de l'eau, des éléments sanguins, des électrolytes, des éléments nutritifs, des médiateurs inflammatoires, des leucocytes, des enzymes protéolytiques, facteurs de croissances, etc. L'exsudat de par sa composition a un effet bénéfique pour le déroulement normal de la cicatrisation des plaies, et doit être maintenu au niveau de la plaie le plus longtemps possible. Cependant, il peut vite devenir un problème majeur pour la plaie et la peau autour de celle-ci lorsque la quantité de fluide produite entraîne des phénomènes de macération qui altèrent le processus de cicatrisation avec des risques infectieux majeurs. En outre, la production abondante d'exsudats peut avoir un impact négatif sur la qualité de vie du patient, sur son état psychologique ou sa vie sociale.

Il serait souhaitable de pouvoir mesurer la quantité d'exsudats d'une plaie pendant une période donnée et suivre l'évolution temporelle des exsudats, de manière à pouvoir proposer des soins et pansements appropriés favorisant le déroulement normal de la cicatrisation de la plaie.

D'une manière traditionnelle, la quantification des exsudats est réalisée en inspectant la plaie et le pourtour cutané de celle-ci afin de repérer l'existence d'éventuels signes de macération ou d'inflammation. Toutefois, cette méthode comporte un certain degré de subjectivité, ne permet pas de suivre objectivement l'évolution temporelle des exsudats et peut mener à une erreur de diagnostic et à une mauvaise prise en charge de la plaie.

Une autre méthode consiste à apprécier le poids et/ou la fréquence de changement de pansements imprégnés d'exsudats pendant une période donnée. Toutefois, cette méthode est difficile à mettre en oeuvre pour le personnel soignant en pratique quotidienne, n'est pas rapide et ne permet pas d'obtenir des résultats fiables.

Ainsi, compte tenu de ce qui précède, l'invention a pour but de proposer un dispositif ou support de quantification permettant de réduire le plus possible la part de subjectivité dans la quantification des exsudats d'une plaie.

Un autre but de l'invention est de proposer un dispositif ou support de quantification permettant d'estimer de manière simple, rapide et objective la quantité d'exsudats d'une plaie pendant une période donnée.

Encore un autre but de l'invention est de proposer un tel dispositif ou support de quantification qui soit d'application simple et économique et qui permette d'estimer la quantité d'exsudats sans toucher la plaie pour éviter tout risque de contamination.

L'invention a également pour but de fournir un procédé qui soit simple, fiable, de mise en oeuvre facile et qui permette d'estimer rapidement la quantité d'exsudats d'une plaie pendant une période donnée en vue de proposer des soins et pansements appropriés favorisant le déroulement normal de la cicatrisation.

Encore un autre but de l'invention est de fournir un tel procédé, qui permette de suivre dans le temps l'évolution des exsudats d'une plaie et de déterminer l'efficacité de la prise en charge de la plaie.

### Divulgation de l'invention

La solution proposée par l'invention est un procédé de quantification des exsudats s'écoulant d'une plaie pendant une période déterminée, de préférence pendant une période de 24 heures. Ce procédé est remarquable en ce qu'il comprend les étapes suivantes :
a) placer :
   ■ un support de quantification des exsudats transparent ou semi-transparent, et comportant un abaque défini par une pluralité de zones (Z1, Z2,... Zn) différentes, la surface de chacune desdites zones correspondant à une quantité déterminée d'exsudats susceptible de s'être écoulée de ladite plaie pendant ladite période,
      sur
   ■ la tache formée par lesdits exsudats sur le pansement absorbant ayant servi à protéger ladite plaie pendant ladite période, de manière à ce que le contour de ladite tache s'inscrive au moins dans la zone de l'abaque qui a la plus petite surface,
b) déduire la quantité d'exsudats susceptible de s'être écoulée de la plaie pendant ladite période en déterminant la zone qui a la plus grande surface dans laquelle ladite tache est inscrite.

Ce procédé présente notamment l'avantage de permettre une estimation objective plus précise que dans l'art antérieur et qui est plus en rapport avec la quantité d'exsudats (réellement) produite pendant une période déterminée, par exemple pendant une période de 24 heures. Il s'agit d'un procédé rapide qui peut être mis en oeuvre sans qu'il soit nécessaire de toucher ou d'entrer en contact intime avec la plaie en question, permettant ainsi d'écarter tout risque de contamination, puisque cette estimation est réalisée de manière indirecte, à partir de la tache formée par les exsudats sur le pansement absorbant ayant servi à protéger la plaie pendant une période déterminée, par exemple pendant une période de 24 heures, ladite tache pouvant avoir une forme générale ronde, plus ou moins régulière, ou une forme générale ovale ou allongée.

Le demandeur a en effet pu montrer par des essais effectués en laboratoire sur de nombreux pansements absorbants différents, qu'il était possible de quantifier les exsudats d'une plaie directement à partir de la tache laissée par ces derniers sur le pansement absorbant ayant servi à protéger ladite plaie, par la mise en oeuvre d'un support de quantification des exsudats particulier. Ce support de quantification particulier qui sera décrit plus en détail ci-dessous, est amovible par rapport au pansement absorbant et présente un abaque donnant pour un pansement absorbant donné (p. ex. pansement hydrocellulaire, hydrocolloïde, alginate, hydrofibre, ou autre) et pour une tache d'exsudats de forme notamment ronde ou ovale, la quantité d'exsudats écoulée d'une plaie pendant une période donnée, en particulier pendant 24 heures.

Certes, on connait de l'art antérieur le document US 2002/0062114 A1 (Murai et al.) qui divulgue une grille à mailles pour estimer la quantité de liquides, notamment des exsudats s'écoulant d'une plaie, la surface de chacune desdites mailles correspondants à une partie seulement de la quantité écoulée de liquides. Cette grille à mailles fait partie intégrante d'un article absorbant tel qu'un pansement, qui comporte une feuille supérieure perméable aux liquides et destinée à être en contact avec la plaie, une feuille de support imperméable aux liquides et un corps absorbant disposé en sandwich entre ladite feuille supérieure et ladite feuille de support. Une telle grille à mailles présente cependant plusieurs inconvénients : d'une part, étant imprimée sur la face externe de la feuille supérieure, elle doit être placée sur le site de production des exsudats en vue d'estimer la quantité d'exsudats, ce que la présente invention cherche à éviter, et d'autre part, faisant partie intégrante d'un article absorbant à usage unique, elle ne peut servir qu'une seule fois contrairement au support de quantification de la présente invention qui peut être utilisé plusieurs fois indépendamment du pansement protégeant la plaie. Par ailleurs, l'estimation d'exsudats à l'aide d'une telle grille à mailles nécessite plusieurs étapes : (i) enlèvement de l'article absorbant protégeant la plaie, (ii) comptage du nombre de mailles couvrant la marque laissée par les exsudats sur ladite grille à mailles (ou ladite feuille supérieure), et enfin (iii) multiplication du nombre de mailles déterminé par la capacité maximale d'absorption du corps absorbant.

D'autres caractéristiques préférées de l'invention sont listées ci-dessous, chacune de ces caractéristiques pouvant être considérée seule ou en combinaison avec les caractéristiques remarquables définies ci-dessus :
- les zones (Z1, Z2,... Zn) du support de quantification mis en oeuvre à l'étape (a) sont des rectangles qui s'inscrivent les uns dans les autres et qui ont la même origine (O).
- l'abaque (2) du support de quantification mis en oeuvre à l'étape (a) est défini par quatre zones (Z1, Z2, Z3, Z4), la surface de chacune desdites zones correspondant à une quantité d'exsudats susceptible de s'écouler de la plaie pendant une période déterminée.
- l'abaque (2) du support de quantification mis en oeuvre à l'étape (a) est associé à un repère (OXY) avec un axe des abscisses (OX) et un axe des ordonnées (OY) et une origine (O) commune auxdits axes (OX) et (OY).
- l'abaque (2) du support de quantification mis en oeuvre à l'étape (a) comprend deux demi-droites (Od1) et (Od2) de positionnement de même origine (O), lesdites demi-droites (Od1) et (Od2), formant respectivement, deux angles distincts θ₁ et θ₂ avec l'axe des abscisses (OX), de préférence, lesdits angles θ₁ et θ₂ mesurent respectivement 25° et 80°.
- lorsque la tache formée par les exsudats a une forme générale ronde, le support de quantification selon l'invention est placé, à l'étape (a), de manière à ce qu'une extrémité du contour de ladite tache touche l'axe des abscisses (OX) ou l'axe des ordonnées (OY).
- lorsque la tache a une forme générale ovale ou allongée, le support de quantification selon l'invention est placé, à l'étape (a), de manière à ce que deux extrémités du contour de ladite tache touchent respectivement les demi-droites de positionnement (Od1 et Od2).
- les étapes (a) et (b) sont répétées autant de fois qu'il est nécessaire, avantageusement environ toutes les 24 heures.

Selon un autre aspect, l'invention a pour objet un support de quantification des exsudats d'une plaie. Ce support de quantification est remarquable par le fait qu'il est transparent ou semi-transparent, et par le fait qu'il comporte un abaque défini par une pluralité de zones (Z1, Z2,... Zn) rectangulaires s'inscrivant les unes dans les autres et ayant la même origine (O), la surface de chacune desdites zones correspondant à une quantité déterminée d'exsudats susceptible de s'être écoulée de ladite plaie pendant ladite période.

Un tel support de quantification est économique tout en contribuant à la préservation de l'environnement car il ne fait partie intégrante d'aucun pansement absorbant et peut être réutilisé à l'infini pour quantifier les exsudats d'une plaie. Il présente également l'avantage d'une grande souplesse d'utilisation puisqu'il peut être (ré)utilisé avec différents types de pansements absorbants (p. ex. hydrocellulaires, hydrocolloïdes, alginates, hydrofibres, etc.). il permet, en outre, une appréciation rapide et objective de la quantité des exsudats écoulés d'une plaie pendant une période donnée, par exemple pendant 24 heures. Il apporte ainsi une aide appréciable à la prise de décision concernant le choix du pansement et/ou du traitement adéquat qui permettra de favoriser le déroulement normal de la cicatrisation.

D'autres caractéristiques préférées du support de quantification de l'invention sont listées ci-dessous, chacune de ces caractéristiques pouvant être considérée seule ou en combinaison avec les caractéristiques remarquables définies ci-dessus :
- L'abaque est défini par quatre zones (Z1, Z2, Z3 et Z4), la surface de chacune desdites zones correspondant à une quantité d'exsudats susceptible de s'écouler de la plaie pendant une période déterminée, de préférence pendant une période de 24 heures.
- Pour une période de 24 heures et pour un pansement absorbant donné, la zone :
   ■ Z1 mesure entre 10 et 20 cm² et correspond à une quantité d'exsudats susceptibles de s'écouler de la plaie allant de 2 à 6 grammes, pour les pansements absorbants de type hydrocellulaires, hydrocolloïdes, ou compresses, à l'exception des pansements alginates, hydrofibres, ou polyacrylates,
   ■ Z2 mesure entre 20 et 35 cm² et correspond à une quantité d'exsudats susceptibles de s'écouler de la plaie allant de 6 à 10 grammes d'exsudats susceptibles de s'écouler de la plaie, pour les pansements absorbants de type hydrocellulaires, à l'exception des pansements alginates, hydrofibres, polyacrylates ou hydrocolloïdes et des compresses,
   ■ Z3 mesure entre 35 et 60 cm² et correspond à une quantité d'exsudats susceptibles de s'écouler de la plaie allant de 10 à 20 grammes, pour les pansements absorbants de types hydrofibres, polyacrylates ou alginates, à l'exception des pansements hydrocolloïdes, des mousses hydrocellulaires et des compresses,
   ■ Z4 mesure 60 à 110 cm² et correspond à une quantité d'exsudats susceptibles de s'écouler de la plaie allant de 20 à 40 grammes, pour les pansements absorbants de types hydrofibres, polyacrylates ou alginates, à l'exception des pansements hydrocolloïdes, des mousses hydrocellulaires et des compresses.
- De préférence, pour une période de 24 heures et pour un pansement absorbant donné, la zone :
   ■ Z1 mesure 13,6 cm² et correspond à environ 4,2 grammes d'exsudats susceptibles de s'écouler de la plaie, pour les pansements absorbants de type hydrocellulaires, hydrocolloïdes ou compresses, à l'exception des pansements alginates, hydrofibres ou polyacrylates,
   ■ Z2 mesure 28,6 cm² et correspond à environ 8,5 grammes d'exsudats susceptibles de s'écouler de la plaie, pour les pansements absorbants de type hydrocellulaires, à l'exception des pansements alginates, hydrofibres, polyacrylates ou hydrocolloïdes et des compresses,
   ■ Z3 mesure 53,3 cm² et correspond à environ 16,0 grammes d'exsudats susceptibles de s'écouler de ladite plaie, pour les pansements absorbants de types hydrofibres, polyacrylates ou alginates, à l'exception des pansements hydrocolloïdes, des mousses hydrocellulaires et des compresses,
   ■ Z4 mesure 94,3 cm² et correspond à environ 28,2 grammes d'exsudats susceptibles de s'écouler de ladite plaie, pour les pansements absorbants de types hydrofibres, polyacrylates ou alginates, à l'exception des pansements hydrocolloïdes, des mousses hydrocellulaires et des compresses.
- L'abaque est associé à un repère (OXY) avec un axe des abscisses (OX) et un axe des ordonnées (OY) et une origine (O) commune auxdits axes (OX) et (OY).
- L'abaque comprend deux demi-droites (Od1) et (Od2) de positionnement de même origine (O), lesdites demi-droites (Od1) et (Od2), formant respectivement, deux angles distincts θ₁ et θ₂ avec l'axe des abscisses (OX), de préférence, lesdits angles θ₁ et θ₂ mesurent respectivement 25° et 80°.

La présente invention vise également l'utilisation d'un tel support de quantification pour l'estimation de la quantité d'exsudats d'une plaie pendant une période déterminée, de préférence pendant une période de 24 heures.

La présente invention concerne aussi un ensemble pansement-support de quantification comportant :
- un pansement absorbant destiné à être placé sur une plaie, pendant une période donnée, par exemple pendant 24 heures pour absorber les exsudats s'écoulant de ladite plaie, puis à être retiré de ladite plaie après ladite période, pour pouvoir procéder à la quantification des exsudats, et
- un support de quantification des exsudats d'une plaie, transparent ou semi-transparent, et comportant un abaque défini par une pluralité de zones (Z1, Z2, Z3,...Zn).

Cet ensemble est remarquable en ce que lesdites zones (Z1, Z2, Z3,...Zn) ont des surfaces différentes, chacune desdites surfaces correspondant à une quantité déterminée d'exsudats susceptible de s'être écoulée de ladite plaie. Il est également remarquable en ce que ledit support de quantification est amovible par rapport audit pansement et est destiné à être positionné sur la face dudit pansement qui a été en contact avec ladite plaie et qui comporte une tache formée par les exsudats, de sorte que le contour de ladite tache s'inscrive au moins dans la zone de l'abaque qui a la plus petite surface.

### Description des figures.

L'invention sera mieux comprise à la lecture de la description qui suit, donnée uniquement à titre d'exemple, et faite en référence aux figures en annexe dans lesquelles :
- la figure 1 est une vue de face du support de quantification selon un mode de réalisation préféré de l'invention.
- la figure 2 illustre une variante du support de quantification de la figure 1.
- La figure 3 illustre un pansement absorbant (3) avec une tache (4) de forme générale ronde, plus ou moins régulière, formée par les exsudats.
- La figure 4 montre un exemple d'estimation de la quantité d'exsudats à partir d'une tache (4) de forme générale ronde, plus ou moins régulière.
- La figure 5 illustre un pansement absorbant (3) avec une tache (4') de forme générale ovale ou allongée formée par les exsudats.
- La figure 6 montre un exemple d'estimation de la quantité d'exsudats à partir d'une tache (4') de forme générale ovale ou allongée.
- La figure 7 est un graphique représentant un suivi de l'évolution des exsudats et sur lequel sont représentés en abscisses les périodes de 24 heures et en ordonnées les différentes zones.

### Modes de réalisation de l'invention

Le support de quantification et le procédé de l'invention permettent d'estimer la quantité d'exsudats d'une plaie en vue d'identifier les soins appropriés pour une prise en charge optimale de la plaie.

Par « plaie » on entend selon l'invention toute lésion de la peau d'un patient humain ou animal, y compris les plaies dues à des accidents et les incisions chirurgicales.

Par « quantification » : on entend selon l'invention l'estimation de la quantité des exsudats s'écoulant d'une plaie pendant une période déterminée, par exemple pendant une période de 24 heures.

Par « pansement absorbant », on entend selon l'invention, tous types de pansements connus de l'homme du métier, comprenant un matériau absorbant et utilisés pour le traitement des plaies, notamment des plaies exsudatives. A titre d'exemple non limitatif d'un tel matériau absorbant on peut citer, les alginates, leurs dérivés, les dérivés de cellulose, les dérivés d'acide polyacrylique ou polyméthacrylique, les dérivés de polyacrylamide, les dérivés d'éther polyvinylique, les gommes, les compresses, ou autres.

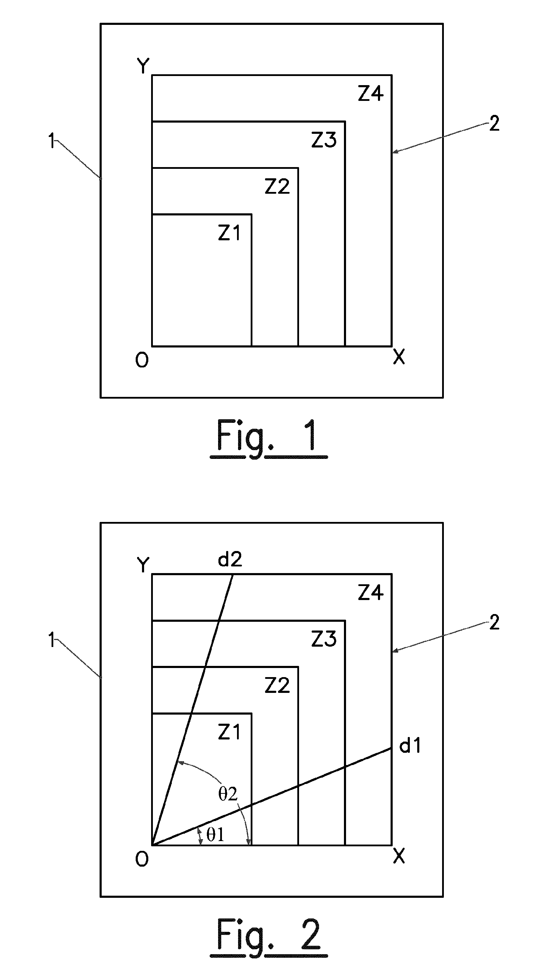

On se rapporte tout d'abord à la figure 1 qui montre un support de quantification (1) des exsudats d'une plaie. Ce support de quantification (1) est avantageusement transparent, semi-transparent ou translucide pour permettre à l'opérateur de voir le contour de la tache formée par les exsudats sur le pansement absorbant et de procéder à l'estimation des exsudats.

Le support de quantification (1) peut être réalisé en matériau rigide, semi-rigide ou flexible, de préférence en matériau semi-rigide ou flexible pour s'adapter à la surface du pansement absorbant.

A titre purement illustratif mais non limitatif, le support de quantification (1) peut être réalisé en polyéthylène, en polypropylène, en chlorure de polyvinyle (PVC) ou en polyester.

Le support de quantification (1) peut avoir toute forme appropriée, notamment être de forme carrée ou rectangulaire.

De manière à permettre l'estimation objective de la quantité d'exsudats d'une plaie, le support de quantification (1) comporte un abaque (2).

L'abaque (2) est défini par une pluralité de zones (Z1, Z2,... Zn), la surface de chacune desdites zones correspondant à une quantité déterminée d'exsudats susceptible de s'être écoulée de la plaie en question pendant une période déterminée, de préférence pendant 24 heures.

En pratique, les zones (Z1, Z2,... Zn) du support de quantification (1) sont des rectangles qui s'inscrivent les uns dans les autres et qui ont la même origine (O).

De préférence, l'abaque (2) est défini par quatre zones (Z1, Z2, Z3 et Z4), la surface de chaque zone correspondant à une quantité d'exsudats susceptible de s'être écoulée de la plaie pendant une période déterminée, de préférence pendant 24 heures.

Un tel abaque (2) a été établi expérimentalement d'après un algorithme qui prend en compte :
■ le coefficient d'absorption (y compris la capacité de rétention des exsudats) du pansement absorbant destiné à être en contact avec la plaie,
■ le poids du pansement mesuré avant et après contact avec une quantité donnée d'eau ou d'exsudats pendant une période déterminée, de préférence pendant 24 heures,
■ et, la surface de la tache formée par la quantité donnée d'eau ou d'exsudats sur le pansement.

Il convient de rappeler que le coefficient d'absorption (appelé aussi propriétés d'absorption ou de drainage) des pansements absorbants, est mesuré conformément à la norme EN 13726-1 (chapitre 3.3). En général, ce coefficient d'absorption est :
■ supérieur ou égal à 0,15 g/cm²/24h pour les pansements hydrocolloïdes,
■ supérieur ou égal à 0,30 g/cm²/24h pour les pansements hydrocellulaires,
■ supérieur ou égal à 6 g/cm²/24h pour les pansements alginates ou polyacrylates,
■ supérieur ou égal à 7 g/cm²/24h pour les pansements hydrofibres,
■ supérieur ou égal à 0,65 g/cm²/24h pour les compresses (ou pièces de gazes hydrophiles tissées, ou de coton non tissé).

Les zones Z1, Z2, Z3 et Z4 de l'abaque (2) ont été dimensionnées de manière à couvrir une large gamme de plaies. A titre d'exemple, les dimensions des zones Z1, Z2, Z3 et Z4 de l'abaque (2) et les quantités d'exsudats correspondantes pour une période déterminée de 24 heures sont les suivantes :
■ Z1 mesure entre 10 et 20 cm², de préférence environ 13,6 cm², et correspond à une quantité allant de 2 à 6 grammes, préférentiellement d'environ 4,2 grammes d'exsudats susceptibles de s'écouler de la plaie, pour les pansements absorbants de type hydrocellulaires, hydrocolloïdes ou compresses, à l'exception des pansements alginates, hydrofibres ou polyacrylates,
■ Z2 mesure entre 20 et 35 cm², de préférence environ 28,6 cm², et correspond à une quantité allant de 6 à 10 grammes, préférentiellement d'environ 8,5 grammes d'exsudats susceptibles de s'écouler de la plaie, pour les pansements absorbants de type hydrocellulaires, à l'exception des pansements alginates, hydrofibres, polyacrylates ou hydrocolloïdes et des compresses,
■ Z3 mesure entre 35 et 60 cm², de préférence environ 53,3 cm², et correspond à une quantité allant de 10 à 20 grammes, préférentiellement d'environ 16,0 grammes d'exsudats susceptibles de s'écouler de ladite plaie, pour les pansements absorbants de types hydrofibres, polyacrylates ou alginates, à l'exception des pansements hydrocolloïdes, des mousses hydrocellulaires et des compresses,
■ Z4 mesure entre 60 et 110 cm², de préférence environ 94,3 cm², et correspond à une quantité allant de 20 à 40 grammes, préférentiellement d'environ 28,2 grammes d'exsudats susceptibles de s'écouler de ladite plaie, pour les pansements absorbants de types hydrofibres, polyacrylates ou alginates, à l'exception des pansements hydrocolloïdes, des mousses hydrocellulaires et des compresses.

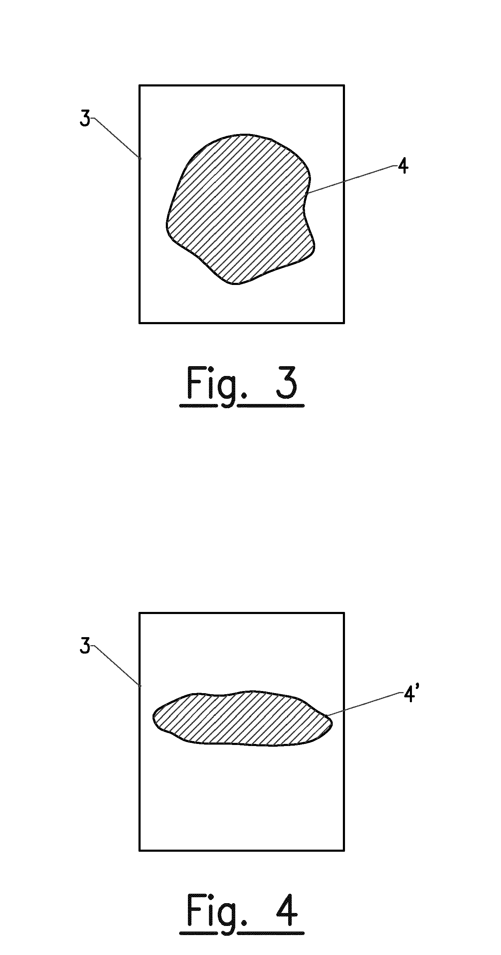

Avantageusement, l'abaque (2) est associé à un repère (OXY) avec un axe des abscisses (OX) et un axe des ordonnées (OY) et une origine (O) commune auxdits axes (OX) et (OY), pour permettre de positionner correctement le support de quantification (1) de l'invention sur la tache (4) formée par les exsudats sur le pansement absorbant. La tache (4) présente généralement une forme ronde, plus ou moins régulière, telle qu'illustré à la figure 3.

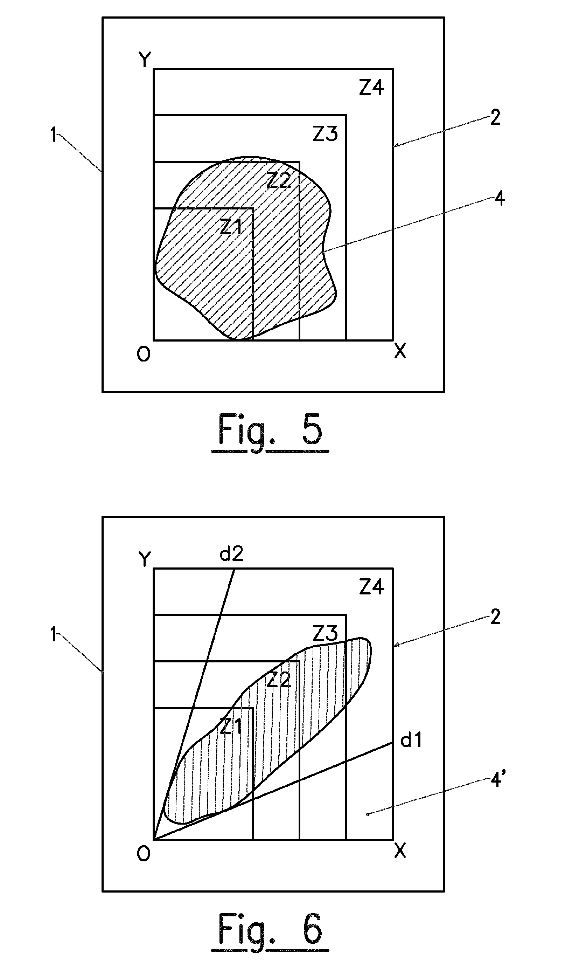

Dans une variante de réalisation, représentée à la figure 2, l'abaque (2) comprend, en outre, deux demi-droites (Od1) et (Od2) de positionnement ayant la même origine (O) et formant, respectivement, un angle θ₁ = 25° et un angle θ₂ = 80° avec l'axe des abscisses (OX). Cette variante a pour avantage de permettre une estimation encore plus précise de la quantité d'exsudats susceptibles de s'être écoulée de la plaie. En particulier, cette variante est intéressante au cas où la tache (4') présente une forme générale ovale ou allongée telle que représentée à la figure 5.

Un autre aspect de l'invention concerne un procédé mettant en oeuvre un support de quantification (1) selon l'invention pour estimer la quantité d'exsudats d'une plaie à partir de la tache (4, 4') formée par les exsudats sur le pansement absorbant (3) ayant servi à protéger la plaie pendant une période déterminée, de préférence pendant 24 heures. Ce procédé comprend les étapes suivantes :
a) placer :
   ■ un support de quantification (1) des exsudats transparent ou semi-transparent, et comportant un abaque (2) défini par une pluralité de zones (Z1, Z2,... Zn) différentes, la surface de chacune desdites zones correspondant à une quantité déterminée d'exsudats susceptible de s'être écoulée de ladite plaie pendant ladite période,
      sur
   ■ la tache (4, 4') formée par lesdits exsudats sur le pansement absorbant (3) ayant servi à protéger ladite plaie pendant ladite période, de manière à ce que le contour de ladite tache (4, 4') s'inscrive au moins dans la zone de l'abaque (2) qui a la plus petite surface,
b) déduire la quantité d'exsudats susceptible de s'être écoulée de la plaie pendant ladite période en déterminant la zone qui a la plus grande surface dans laquelle ladite tache (4, 4') est inscrite.

Lorsque la tache (4) a une forme générale ronde, plus ou moins régulière, le dispositif conforme à la présente invention peut être positionné de manière à ce qu'une extrémité du contour de la tache (4) touche l'axe des abscisses (OX) ou l'axe des ordonnées (OY).

Lorsque la tache (4') a une forme générale ovale ou allongée, le support de quantification (1) conforme à la variante représentée à la figure 2, peut être positionné de manière à ce que deux extrémités du contour de la tache (4') touchent respectivement les demi-droites de positionnement (Od1) et (Od2).

Le procédé selon l'invention peut être répété autant de fois qu'il est nécessaire, avantageusement environ toutes les 24 heures, avant de prendre une décision songée quant au choix du pansement et/ou du traitement adapté pour la plaie en question.

Le support de quantification (1) de la présente l'invention s'est révélé très fiable pour déduire rapidement et objectivement la quantité d'exsudats d'une plaie ainsi que pour suivre l'évolution temporelle des exsudats et par conséquent pour déterminer l'efficacité de la prise en charge de la plaie en question.

Aussi, l'invention a encore pour objet l'utilisation d'un tel support de quantification (1) pour l'estimation de la quantité d'exsudats d'une plaie pendant une période donnée, par exemple pendant 24 heures.

À titre d'exemple non limitatif, la figure 5 représente un support de quantification (1) selon l'invention placé sur une tache (4) de forme générale ronde, plus ou moins régulière, qui a été formée par les exsudats sur un pansement absorbant de type hydrofibres ayant servi à protéger la plaie aiguë d'un patient pendant une période de 24 heures. Sur cette figure, on peut voir que le contour de la tache (4) est inscrit dans la zone Z3 de l'abaque (2). Connaissant le type de pansement absorbant utilisé, et sachant que la zone Z3 présente une surface comprise entre 35 et 60 cm², de préférence égale à environ 53,3 cm², on peut en déduire que la quantité d'exsudats susceptible de s'être écoulée de la plaie pendant une période de 24 heures est comprise entre 10 à 20 grammes, préférentiellement est égale à environ 16,0 grammes.

Un autre exemple d'estimation de la quantité d'exsudats est illustré par la figure 6 qui montre un support de quantification (1) selon une variante de l'invention placé sur une tache (4') de forme générale ovale ou allongée formée par les exsudats sur un pansement absorbant de type hydrofibres ayant servi à protéger la plaie d'un patient pendant une période de 24 heures. Sur cette figure, on peut voir que le dispositif selon cette variante est placé de manière à ce que deux extrémités du contour de la tache (4') touchent la demi-droite (Od1) et une autre extrémité dudit contour touche la demi-droite (Od2). D'après cet exemple, le contour de la tache (4') est inscrit dans la zone Z4 de l'abaque (2). Connaissant le type de pansement absorbant utilisé (ici un pansement hydrofibre), et sachant que la zone Z4 présente une surface de 60 à 110 cm², on peut en déduire que la quantité d'exsudats susceptible de s'être écoulée de la plaie pendant une période de 24 heures est d'environ 20 à 40 grammes.

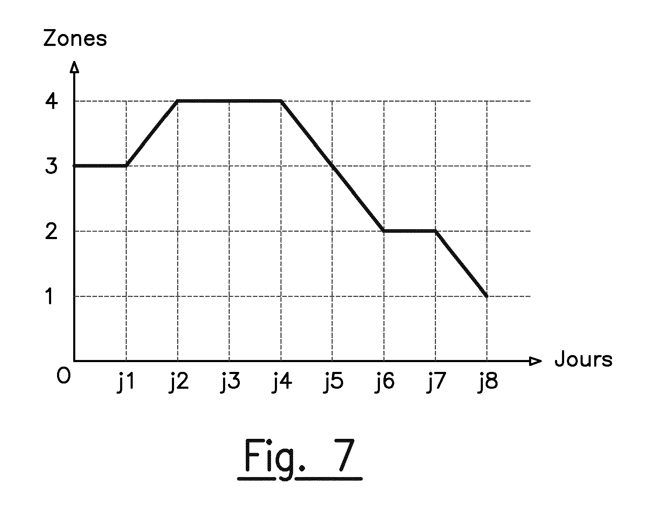

En référence à la figure 7, il est présenté un graphe de suivi de l'évolution des exsudats pour la même plaie que dans l'exemple illustré par la figure 4. Le pansement absorbant (3) de type hydrofibre a été changé une fois par jours à J0, J1, J2, J3, J4, J5, J6, J7 et J8.

La zone correspondant à la tache (4) formée sur chaque pansement a été relevée quotidiennement, pendant 8 jours.

L'analyse de la courbe représentée sur la figure 7 permet de voir qu'entre J0 et J4, la zone correspondant à la tache (4) est passée de Z3 à Z4, ce qui signifie que la production des exsudats a été importante entre J0 et J4. Dans ce cas, un pansement absorbant de type hydrofibres, polyacrylates ou alginates peut être proposé en tant que soin pour favoriser la cicatrisation de cette plaie.

En revanche, entre J4 et J8, la zone correspondant à la tache (4) est passée de Z4 à Z1, ce qui signifie que la production d'exsudats est devenue moins importante entre J4 et J8. Dans ce cas un pansement moins absorbant, par exemple un pansement mousse peut être proposé en vue de maintenir un taux d'humidité suffisant au sein de la plaie pour une cicatrisation dans de bonnes conditions.

L'invention permet aussi de faire en sorte que le support de quantification conforme à l'invention et au moins un pansement absorbant soient conditionnés dans un seul emballage approprié, transparent ou opaque, notamment stérile et imperméable à l'eau, pour leur vente et leur conservation. Aussi, l'invention vise également un ensemble pansement-support de quantification comportant :
- un pansement absorbant destiné à être placé sur une plaie, pendant une période donnée, par exemple pendant 24 heures pour absorber les exsudats s'écoulant de ladite plaie, puis à être retiré de ladite plaie après ladite période, pour pouvoir procéder à la quantification des exsudats, et
- un support de quantification des exsudats d'une plaie, transparent ou semi-transparent, et comportant un abaque défini par une pluralité de zones (Z1, Z2, Z3,...Zn).

Cet ensemble pansement-support de quantification se caractérise par le fait que les zones (Z1, Z2, Z3,...Zn) du support de quantification ont des surfaces différentes, chacune desdites surfaces correspondant à une quantité déterminée d'exsudats susceptible de s'être écoulée de ladite plaie. Un tel ensemble se caractérise également par le fait que le support de quantification est amovible par rapport au pansement absorbant et est destiné à être positionné sur la face du pansement qui a été en contact avec la plaie et qui comporte une tache formée par les exsudats, de manière à ce que le contour de ladite tache s'inscrive au moins dans la zone de l'abaque qui a la plus petite surface.

Il convient de noter que l'ensemble selon l'invention peut comporter deux ou plusieurs pansements absorbants de même type ou de type différent. A titre d'exemple non limitatif de pansement absorbant pouvant être utilisé avec le support de quantification selon l'invention, sauf indication contraire, on peut citer : les hydrocellulaires, les hydrocolloïdes, les alginates, les hydrofibres, les polyacrylates, les compresses ou autres.

## Revendications

1. Procédé de quantification des exsudats s'écoulant d'une plaie pendant une période déterminée, comprenant les étapes suivantes :
a) placer :
- un support de quantification (1) des exsudats transparent ou semi-transparent, et comportant un abaque (2) défini par une pluralité de zones (Z1, Z2,... Zn) **différentes,** la surface de chacune desdites zones correspondant à une quantité déterminée d'exsudats susceptible de s'être écoulée de ladite plaie pendant ladite période,
sur
- la tache (4, 4') formée par lesdits exsudats sur le pansement absorbant (3) ayant servi à protéger ladite plaie pendant ladite période, de manière à ce que le contour de ladite tache (4, 4') s'inscrive au moins dans la zone de l'abaque (2) qui a la plus petite surface,
b) déduire la quantité d'exsudats susceptible de s'être écoulée de la plaie pendant ladite période en déterminant la zone qui a la plus grande surface dans laquelle ladite tache (4, 4') est inscrite.

2. Procédé selon la revendication 1, **caractérisé en ce qu'à** l'étape (a) le support de quantification (1) est placé sur la face du pansement qui n'a pas été directement en contact avec la plaie.

3. Procédé selon la revendication 1 ou 2, **caractérisé en ce que** les zones (Z1, Z2,... Zn) du support de quantification mis en oeuvre à l'étape (a) sont des rectangles qui s'inscrivent les uns dans les autres et qui ont la même origine (O).

4. Procédé selon l'une des revendications précédentes, **caractérisé en ce que** l'abaque (2) du support de quantification mis en oeuvre à l'étape (a) est défini par quatre zones (Z1, Z2, Z3, Z4), la surface de chacune desdites zones correspondant à une quantité d'exsudats susceptible de s'écouler de la plaie pendant une période déterminée.

5. Procédé selon l'une des revendications précédentes, **caractérisé en ce que** l'abaque (2) du support de quantification mis en oeuvre à l'étape (a) est associé à un repère (OXY) avec un axe des abscisses (OX) et un axe des ordonnées (OY) et une origine (O) commune auxdits axes (OX) et (OY).

6. Procédé selon l'une des revendications précédentes, **caractérisé en ce que** l'abaque (2) du support de quantification mis en oeuvre à l'étape (a) comprend deux demi-droites (Od1) et (Od2) de positionnement de même origine (O), lesdites demi-droites (Od1) et (Od2), formant respectivement, deux angles distincts θ₁ et θ₂ avec l'axe des abscisses (OX), de préférence, lesdits angles θ₁ et θ₂ mesurent respectivement 25° et 80°.

7. Procédé selon l'une des revendications précédentes, **caractérisé en ce que** lorsque la tache formée par les exsudats a une forme générale ronde, le dispositif selon l'invention est placé, à l'étape (a), de manière à ce qu'une extrémité du contour de ladite tache touche l'axe des abscisses (OX) ou l'axe des ordonnées (OY).

8. Procédé selon l'une des revendications précédentes, **caractérisé en ce que** lorsque la tache a une forme générale ovale ou allongée, le dispositif selon l'invention est placé, à l'étape (a), de manière à ce que deux extrémités du contour de ladite tache touchent respectivement les demi-droites de positionnement (Od1, Od2).

9. Procédé selon l'une des revendications précédentes, **caractérisé en ce que** les étapes (a) et (b) sont répétées autant de fois qu'il est nécessaire, avantageusement environ toutes les 24 heures.

10. Support de quantification (1) des exsudats d'une plaie **caractérisé en ce qu'il** est transparent ou semi-transparent, et **en ce qu'il** comporte un abaque (2) défini par une pluralité de zones (Z1, Z2,... Zn) rectangulaires s'inscrivant les unes dans les autres et ayant la même origine (O), la surface de chacune desdites zones correspondant à une quantité déterminée d'exsudats susceptible de s'être écoulée de ladite plaie pendant ladite période.

11. Support de quantification (1) selon la revendication 10, **caractérisé en ce que** l'abaque (2) est défini par quatre zones (Z1, Z2, Z3, Z4), la surface de chacune desdites zones correspondant à une quantité d'exsudats susceptible de s'écouler de la plaie pendant une période déterminée.

12. Support de quantification selon la revendication 10 ou 11, **caractérisé en ce que** pour une période de 24 heures et pour un pansement absorbant donné, la zone :
■ Z1 mesure entre 10 et 20 cm² et correspond à une quantité d'exsudats susceptible de s'écouler de la plaie allant de 2 à 6 grammes, pour les pansements absorbants de type hydrocellulaires, hydrocolloïdes, ou compresses, à l'exception des pansements alginates, hydrofibres, ou polyacrylates,
■ Z2 mesure entre 20 et 35 cm² et correspond à une quantité d'exsudats susceptible de s'écouler de la plaie allant de 6 à 10 grammes d'exsudats susceptibles de s'écouler de la plaie, pour les pansements absorbants de type hydrocellulaires, à l'exception des pansements alginates, hydrofibres, polyacrylates ou hydrocolloïdes et des compresses,
■ Z3 mesure entre 35 et 60 cm² et correspond à une quantité d'exsudats susceptibles de s'écouler de la plaie allant de 10 à 20 grammes, pour les pansements absorbants de types hydrofibres, polyacrylates ou alginates, à l'exception des pansements hydrocolloïdes, des mousses hydrocellulaires et des compresses,
■ Z4 mesure 60 à 110 cm² et correspond à une quantité d'exsudats susceptibles de s'écouler de la plaie allant de 20 à 40 grammes, pour les pansements absorbants de types hydrofibres, polyacrylates ou alginates, à l'exception des pansements hydrocolloïdes, des mousses hydrocellulaires et des compresses.

13. Support de quantification selon l'une des revendications 10 à 12, **caractérisé en ce que** pour une période de 24 heures et pour un pansement absorbant donné, la zone :
■ Z1 mesure environ 13,6 cm² et correspond à environ 4,2 grammes d'exsudats susceptibles de s'écouler de la plaie, pour les pansements absorbants de type hydrocellulaires, hydrocolloïdes ou compresses, à l'exception des pansements alginates, hydrofibres ou polyacrylates,
■ Z2 mesure environ 28,6 cm² et correspond à environ 8,5 grammes d'exsudats susceptibles de s'écouler de la plaie, pour les pansements absorbants de type hydrocellulaires, à l'exception des pansements alginates, hydrofibres, polyacrylates ou hydrocolloïdes et des compresses,
■ Z3 mesure environ 53,3 cm² et correspond à environ 16,0 grammes d'exsudats susceptibles de s'écouler de ladite plaie, pour les pansements absorbants de types hydrofibres, polyacrylates ou alginates, à l'exception des pansements hydrocolloïdes, des mousses hydrocellulaires et des compresses.
■ Z4 mesure environ 94,3 cm² et correspond à environ 28,2 grammes d'exsudats susceptibles de s'écouler de ladite plaie, pour les pansements absorbants de types hydrofibres, polyacrylates ou alginates, à l'exception des pansements hydrocolloïdes, des mousses hydrocellulaires et des compresses.

14. Support de quantification selon l'une des revendications 10 à 13, **caractérisé en ce que** l'abaque (2) est associé à un repère (OXY) avec un axe des abscisses (OX) et un axe des ordonnées (OY) et une origine (O) commune auxdits axes (OX) et (OY).

15. Support de quantification selon l'une des revendications 10 à 14, **caractérisé en ce que** l'abaque (2) comprend deux demi-droites (Od1) et (Od2) de positionnement de même origine (O), lesdites demi-droites (Od1) et (Od2), formant respectivement, deux angles distincts θ₁ et θ₂ avec l'axe des abscisses (OX), de préférence, lesdits angles θ₁ et θ₂ mesurent respectivement 25° et 80°.

16. Utilisation d'un support de quantification selon l'une des revendications 10 à 15, pour l'estimation de la quantité d'exsudats d'une plaie pendant une période déterminée, de préférence pendant une période de 24 heures.

17. Ensemble pansement-support de quantification comportant :
- un pansement absorbant destiné à être placé sur une plaie pendant une période donnée pour absorber les exsudats s'écoulant de ladite plaie, puis à être retiré de ladite plaie après ladite période pour pouvoir procéder à la quantification des exsudats, et
- un support de quantification des exsudats d'une plaie, transparent ou semi-transparent, et comportant un abaque défini par une pluralité de zones (Z1, Z2, Z3,...Zn),
ledit ensemble **étant caractérisé par le fait que** lesdites zones (Z1, Z2, Z3,...Zn) ont des surfaces différentes, chacune desdites surfaces correspondant à une quantité déterminée d'exsudats susceptible de s'être écoulée de ladite plaie, **et par le fait que** ledit support de quantification est amovible par rapport audit pansement et est destiné à être positionné sur la face dudit pansement qui a été en contact avec ladite plaie et qui comporte une tache formée par les exsudats, de sorte que le contour de ladite tache s'inscrive au moins dans la zone de l'abaque qui a la plus petite surface.
